# NEW EUROPEAN PATENT SPECIFICATION

(11) **EP 0 183 776 B2**
(45) Date of publication and mention of the opposition decision: **13.09.2000**
(45) Mention of the grant of the patent: 02.01.1992
(21) Application number: 85902779.9
(22) Date of filing: 08.05.1985
(51) Int. Cl.: C12P 21/00, C12N 15/00

(54) **A process for isolating and activating a substantially insoluble polypeptide using non-ionic detergents.**
Verfahren zur Herstellung und Aktivierung eines im wesentlichen nichtlöslichen Polypetids unter Verwendung nichtionischer Detergentien.
Procédé pour la production et l'activation d'une polypetide éssentiellement insoluble en utilisant des détergents non-ioniques.

(30) Priority: 11.05.1984 US 609495
(43) Date of publication of application: 11.06.1986
(73) Proprietor: BERLEX LABORATORIES, INC., Cedar Knolls, NJ 07927-2094 (US)
(72) Inventor: McCAMAN, Michael, T., San Bruno, CA 94066 (US); KING, John, F., Berkeley, CA 94702 (US)
(74) Representative: Thomson, Paul Anthony
(86) International application number: US8500858
(87) International publication number: WO8505377

(56) References cited:
- EP-A- 0 123 928
- EP-A- 0 137 710

## Description

The present invention relates to a process for isolating a substantially insoluble polypeptide or protein using non-ionic detergents. The process is utilisable in relation to the restoration of biological activity to inactive polypeptides or proteins, that is solubilizing, renaturing and restoring activity to proteins which have been partially denatured or inactivated, e.g. during their synthesis in a host cell, such as e.g. E. coli, or during isolation.

With the advent of recombinant DNA technology, it is now possible to introduce foreign genes into microorganisms and regulate the level of their expression.

However, the synthesis of an excess of polypeptide or protein not endogenous to the bacterium may have an adverse effect on the organism's viability, especially if produced at a high level relative to other host proteins. In some instances, the host organism may have a mechanism of disposing of the cloned gene product, e.g. by degrading it or secreting it, thus avoiding interaction with the essential metabolic machinery of the cell. Alternatively, the cloned gene product may assume an altered conformation, thus limiting its interference with normal cellular processes. Disruption or alteration of normal translational or post-translational events, the specific codon usage pattern during protein synthesis,and or host protein-cloned gene product interactions within the cell, may also effect the polypeptide's normal folding and assembly and may result in an inactive conformation of the protein. In a host organism such as E. coli it has been observed in several cases that foreign proteins fold unnaturally and precipitate within the cell to form large inactive protein aggregates, characterized as inclusion bodies (Williams, D.C., et al. Science 215: 687-689 (1982) and Kleid, D.G., et al . Science 214: 1125-1128 (1981)).

It has been shown that native (active) proteins can undergo reversible denaturation (Anfinsen, C., Science 1091:223-230 (1973) and London, J. et al., Eur. J. Biochemistry 47: 409-415 (1974)). The protein is denatured by addition of 8 molar urea and polypeptide refolding occurs spontaneously as the urea is removed. Alkali solutions have also been used as protein solubilizing agents and in some cases can reversibly denature native proteins during brief incubation times (McPhie, P.J. of Biol. Chem 257:689-693 (1982)).

Urea has also been used to aid in the renaturation of the gene product of a recombinant DNA expression system (Emtage, J.S. et al., Proc. Nat. Acad. Sci. USA 80:3671-3675 (1983)).

According to the invention there is provided a process for isolating a substantially insoluble polypeptide being produced by a genetically engineered microorganism which does not naturally produce said gene product, characterised in that the process comprising the steps of:
a) preparing a lysate from the microorganism;
b) reducing the viscosity of the lysate by physical methods;
c) contacting substantially insoluble polypeptide contained in said lysate with a solution containing a non-ionic detergent at a concentration sufficient to solubize impurities while maintaining the insolubility of the polypeptide; and
d) separating the insoluble polypeptide from the soluble impurities;
   with the proviso that the insoluble polypeptide cannot be selected from chymosin, precursor of chymosin and fusion products thereof capable of displaying milk clotting activity.

Characteristically, the polypeptide which is isolated from its host cell, e.g., E. coli, differs in its physical and chemical characteristics from the same polypeptide isolated from its natural environment (calf stomach prochymosin). The same physical and chemical characteristics of the isolated polypeptides are caused to duplicate those of the naturally occurring product by solubilizing the inactive or denatured gene product in a suitable solubilizing reagent and removing the reagent in a manner which allows the polypeptide to assume a thermodynamically stable form possessing its natural biological activity.

A recombinant DNA expression system, and its host microorganism described as JM83/pLC7 was deposited with the American Type Culture Collection, Rockville, Maryland and assigned the accession number ATCC 39325.

In the drawings:

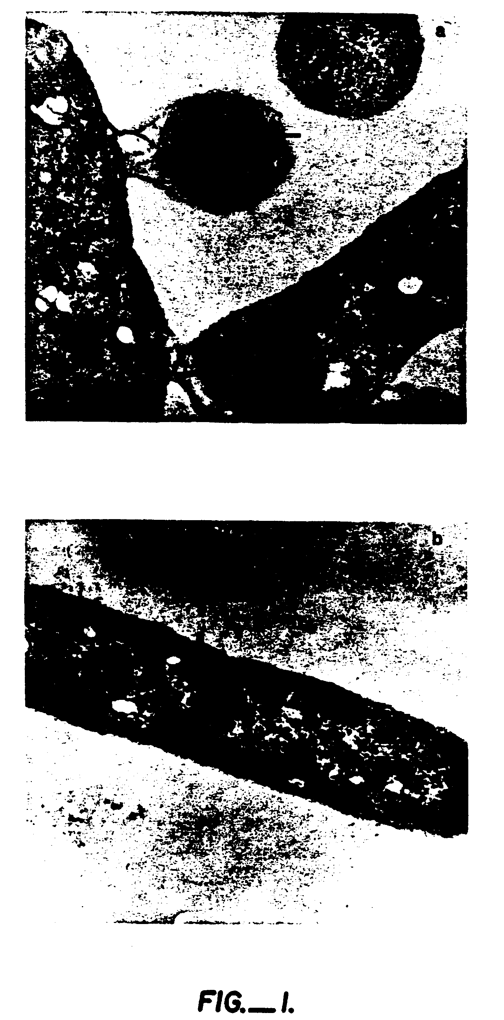

Figure 1 shows an electron micrograph cross-section of E. coli cells (a) containing prochymosin inclusion bodies (arrows) and (b) wild type bacteria which do not contain inclusion bodies.

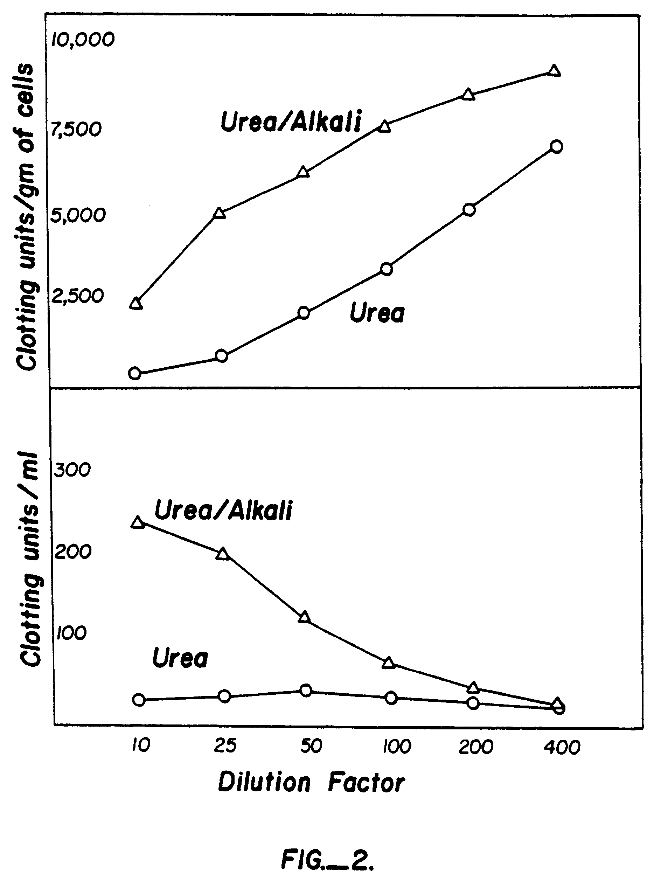

Figure 2 shows the recovery of milk clotting activity derived from pWHA49 prochymosin after solubilization and renaturation by two of the processes disclosed in this invention. The observed activity per volume is plotted against the dilution factor (material from 1 gram of cell paste/final volume in mls) at which the renaturation reaction was performed.

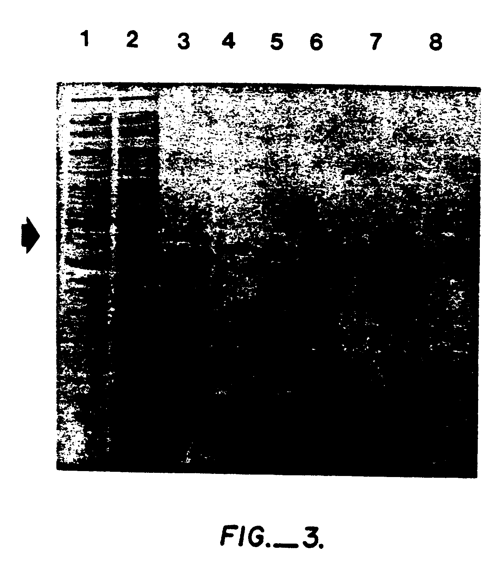

Figure 3 shows a polyacrylamide gel containing protein samples derived from E. coli synthesizing prochymosin, wherein the samples include; total cell proteins from parental E. coli strain CY15001 (lane 1); total cell proteins from prochymosin expression strain CY15001/pWHA49 (lane 2); purified and renatured pWHA49 prochymosin (lane 3); purified and renatured pWHA49 prochymosin after pH 2.0 activation (lane 4); purified and renatured pWHA49 prochymosin after pH 4.5 activation (lane 5); purified calf prochymosin (lane 6); purified calf prochymosin after activation at pH 2.0 (lane 7); and purified calf prochymosin after activation at pH 4.5 (lane 8).

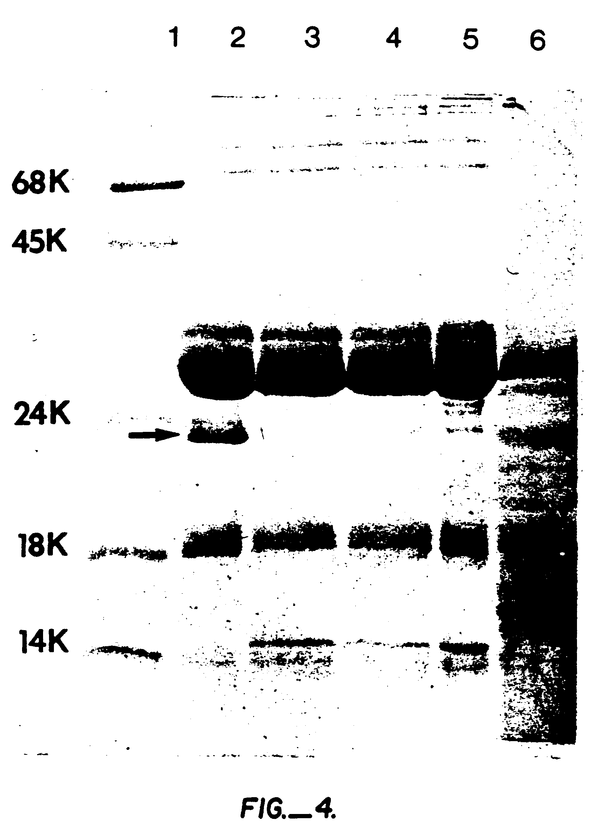

Figure 4 shows a polyacrylamide gel containing protein samples derived from a milk substrate before and after reaction with chymosin derived from either calf or pWHA49 prochymosin, wherein the samples include: molecular weight markers (lane 1); unreacted milk substrate (lane 2); milk coagulated with pWHA49 pseudochymosin (lane 3); milk coagulated with pWHA49 chymosin (lane 4); milk coagulated with calf chymosin (lane 5); and milk substrate coagulated with trypsin (lane 6). The arrow indicates k-casein protein component cleaved by chymosin to initiate coagulation reaction.

The process of the present invention is utilised in relation to the production of active chymosin from a chymosin precursor protein isolated in an insoluble, inactive and/or partially denatured state from E. coli (see Figure 1). The term "denatured" is intended to describe the form of the protein whose conformation and biological activity differs significantly from the same protein isolated from its natural environment. The details of the process are described below.

A bacterial cell paste is resuspended in a buffer containing lysozyme and as cell lysis occurs the viscosity of the solution increases due to the bacterial DNA. For the present invention, it is important to reduce the viscosity of the lysate by physical methods such as sonic disruption of the DNA or use of a French press. The insoluble fraction of the cell is isolated by low speed centrifugation. The insoluble fraction is resuspended in buffer and mixed with a solution containing Triton® X-100 (a non-ionic detergent) for 2-20 hours. This detergent does not solubilize the chymosin precursor protein but does solubilize other bacterial protein contaminants. These contaminants are separated and removed from the insoluble chymosin precursor protein by centrifugation. The insoluble fraction is solubilized in 8M urea for 2-20 hours, preferably 10-16 hours and then diluted into an alkaline phosphate buffer preferably pH 10.5-11.5, and most preferably pH 11.0, incubated for 5-30 minutes, most preferably 10 minutes, and then neutralized slowly to a pH of 8-9, preferably 8.3 and allowed to stand for one hour or more. Subsequent acidification to a pH less than 5 resulted in the generation of a milk clotting activity whose properties are the same as calf stomach chymosin.

The present invention requires several specific steps to achieve efficient recoveries of active chymosin. First, the combination of sonication (after cell lysis) and Triton® X-100 extraction (after collecting the insoluble proteins by centrifugation) are essential to this process.

Table I, infra, illustrates the relative effectivesness in recovery of active chymosin from various precursor renaturation procedures.

**Table I**

| Chymosin Recovery After Various Precursor Renaturation Schemes | | |
|---|---|---|
| | Disruption Method | |
| | Sonication | Enzyme Treatment |
| Insoluble fraction from cell lysate | 7% | - |
| Buffer washed insoluble fraction | 1% | 36% |
| Insoluble fraction after extraction with Triton® X-100 | 100% | 31% |
| Insoluble fraction after extraction with Tween® 20 | 1% | - |

As seen in Table I only low levels of active product (less than 10% of maximal) can be recovered if the Triton® X-100 extraction is omitted. The substitution of another ™non-ionic detergent, Tween®-20, does not facilitate the recovery of high levels of active product. Reducing the vicosity of the initial cell lysate by enzymatic digestion of the bacterial DNA with pancreatic DNase results in low yields of activity from the chymosin precursor protein after its activation and these product yields are not improved by a Triton® X-100 extraction step. Thus, the combination of physical disruption of the bacterial lysate (by sonication) followed by extraction with Triton® X-100 is a novel and unexpected requirement for the present invention.

The second essential feature of this process is the combined use of urea and alkaline pH buffer to solubilize and renature the chymosin precursor protein. Complete solubilization of the insoluble chymosin precursor protein can occur in 8M urea or alkali alone. However, the use of each of these reagents alone have several disadvantages. Solubilization with urea is very rapid but renaturation from urea requires a dialysis step which is very slow. Solubilization with alkali is slower than with urea and is complicated by an irreversible inactivation of the protein which begins after 20 minutes of incubation at pH 11.0 which is approximately the time required for complete solubilization of the chymosin precursor protein. Rapid renaturation from alkaline pH can be achieved by acidification. By simply combining these two procedures we observed a significant improvement in chymosin activity recovered in the final product (see Figure 2). The rapid solubilization in urea is combined with dilution in an alkaline pH buffer and rapid renaturation occurs by a pH neutralization.

The successful renaturation of purified calf prochymosin is dependent on prochymosin concentration during the renaturation reaction. Likewise, a more efficient recovery of active chymosin from the insoluble chymosin precursor isolated from E. coli is dependent on the extent of dilution of the urea solubilized material into the alkaline pH buffer (see Figure 4). The maximal level of chymosin activity achieved by the present invention is greater than or equal to 80% of the predicted limit based upon our estimates of chymosin precursor protein levels in the E. coli lysate and the known specific activity of calf stomach chymosin.

### Experimental

A plasmid pWHA49 was constructed which contains a eukaryotic structural gene coding for prochymosin. This plasmid was used to transform E. coli using known techniques.

10g of frozen cell paste of E. coli strain CY15001 containing pWHA49 were suspended in 100 mls of 25 mm Tris-HCl pH 8, 10 mm EDTA, 1 mg/ml lysozyme. After a short incubation, the lysed cells were further disrupted by sonication. Partial purification of the pWHA49 encoded prochymosin protein was effected by centrifugation at 10,000 x g for ten minutes, followed by an overnight detergent extraction of the pelleted cell debris with 8 percent final concentration of Triton X-100 detergent (Sigma Chemical Co.). The pWHA49 prochymosin protein remained in the insoluble, particulate pellet after the procedure.

Authentic calf prochymosin is a soluble enzyme which can be rapidly activated by acid treatment to a form which efficiently coagulates milk. The E. coli synthesized pWHA49 prochymosin, isolated as described, was insoluble and showed no enzymatic activity after acid treatment.

The pWHA49 prochymosin pellet was suspended in 6.3 mls of 10 mM sodium phosphate buffer at pH 7.5. The suspension is fully solubilized by the addition of solid urea to a final concentration of 6-8 M and then mixed for 16 hours at room temperature.

The resultant clear solution was diluted into 100 volumes (1000 mls) of 25 mM sodium phosphate buffer at pH 11.0, mixed thoroughly and allowed to stand for 10 minutes at room temperature. The pH of the solution was then titrated to pH 8.3 by addition of 0.2M HCl over a period of 3 minutes.

The resultant solution was left at room temperature for one hour or more, after which time acidification to a pH greater than 1.5 and less than 5.0 generated a chymosin milk clotting activity. Acid activation of native calf prochymosin and of pWHA49 prochymosin resulted in the same size proteins, that is, treatment at pH 2 produced pseudochymosin having a molecular weight of approximately 38,500 and treatment at pH 4.5 produced chymosin at a molecular weight of 36,000 (see Figure 3). The renatured, activated product has 50-95 percent of the specific activity of natural calf chymosin and has the same limited substrate specificity (see Figure 4).

The process utilizing urea and alkali demonstrated improvements in renaturing prochymosin, when compared to methods using a single reagent (see Figure 2).

## Claims

1. A process for isolating and activating a substantially insoluble polypeptide being produced by a genetically engineered micro-organism which does not naturally produce said gene product
characterised in that the process comprises the steps of:
(a) preparing a lysate from the micro-organism;
(b) reducing the viscosity of the lysate by physical methods
(c) contacting the substantially insoluble polypeptide contained in the said treated lysate with a solution containing a non-ionic detergent at a concentration sufficient to solubilize impurities while maintaining the insolubility of the polypeptide;
(d) separating the insoluble polypeptide from the soluble impurities;
(e) the insoluble polypeptide after separation from soluble impurities is solubilized by contact with a chaotropic agent ; and
(f) the solubilized polypeptide is activated by removal of the chaotropic agent.
and further characterised in that said non-ionic detergent comprises Triton X-100
with the proviso that the insoluble polypeptide cannot be selected from chymosin, precursors of chymosin and fusion products thereof capable of displaying milk-clotting activity.

2. A process as claimed in claim 1, characterised in that said microorganism is Escherichia coli.

3. A process as claimed in claim 1 or 2, characterised in that the chaotropic agent is urea.

## Patentansprüche

1. Verfahren zum Isolieren und Aktivieren eines im wesentlichen unlöslichen Polypeptids, das von einem genetisch manipulierten Mikroorganismus erzeugt wird, der natürlicherweise dieses Genprodukt nicht herstellt, **dadurch gekennzeichnet**, daß das Verfahren die folgenden Schritte umfaßt:
a) Herstellen eines Lysats aus dem Mikroorganismus
b) Verringern der Viskosität des Lysats durch physikalische Methoden
c) Inkontaktbringen des im wesentlichen unlöslichen Polypeptids, das in dem behandelten Lysat enthalten ist, mit einer Lösung, enthaltend ein nichtionisches Detergenz mit einer Konzentration, die ausreicht, Verunreinigungen zu lösen, während die Unlöslichkeit des Polypeptids beibehalten wird;
d) Abtrennen des unlöslichen Polypeptids von den löslichen Verunreinigungen;
e) das unlösliche Polypeptid wird nach Abtrennung von löslichen Verunreinigungen löslich gemacht durch Kontakt mit einem chaotropen Agenz; und
f) das löslich gemachte Polypeptid wird durch Entfernung des chaotropen Agenzes aktiviert;
und außerdem **dadurch gekennzeichnet**, daß das nichtionische Detergenz Triton X-100 umfaßt,
mit der Maßgabe, daß das unlösliche Polypeptid nicht ausgewählt werden kann aus Chymosin, Vorläufern von Chymosin und Fusionsprodukten davon, die in der Lage sind, Milchgerinnungsaktivität zu zeigen.

2. Verfahren wie in Anspruch 1 beansprucht, **dadurch gekennzeichnet**, daß der Mikroorganismus Escherichia coli ist.

3. Verfahren wie in Anspruch 1 oder 2 beansprucht, **dadurch gekennzeichnet**, daß das chaotrope Agenz Harnstoff ist.

## Revendications

1. Procédé d'isolement et d'activation d'un polypeptide pratiquement insoluble, produit par un microorganisme de génie génétique, qui ne produit pas naturellement ledit produit génique, caractérisé par le fait que le procédé comprend les étapes de :
(a) préparation d'un lysat à partir du microorganisme ;
(b) réduction de la viscosité du lysat par des procédés physiques ;
(c) mise en contact du polypeptide pratiquement insoluble présent dans ledit lysat traité, avec une solution contenant un détergent non-ionique à une concentration suffisante pour solubiliser les impuretés, tout en maintenant l'insolubilité du polypeptide ;
(d) séparation du polypeptide insoluble des impuretés solubles ;
(e) solubilisation du polypeptide insoluble, après sa séparation des impuretés solubles, par mise en contact avec un agent chaotrope ; et
(f) activation du polypeptide solubilisé, par élimination de l'agent chaotrope ;
et caractérisé an outre par le fait que ledit détergent non-ionique comprend du Triton X-100,
à la condition que le polypeptide insoluble ne peut pas être choisi parmi la chymosine, les précurseurs de chymosine et leurs produits de fusion, capables de présenter une activité de coagulation du lait.

2. Procédé selon la revendication 1, caractérisé par le fait que ledit microorganisme est Escherichia coli.

3. Procédé selon la revendication 1 ou 2, caractérisé par le fait que l'agent chaotrope est l'urée.
